# FASCICULE DE BREVET EUROPEEN

(11) **EP 0 582 508 B1**
(45) Date de publication et mention de la délivrance du brevet: **19.11.1997**
(21) Numéro de dépôt: 93401965.4
(22) Date de dépôt: 29.07.1993
(51) Int. Cl.: A61N 5/10

(54) **Procédé de contrôle du faisceau et appareil pour le traitement de lésions par rayonnement à haute énergie**
Verfahren zur Strahlregulierung und Gerät zur Behandlung von Schädigungen mittels hoch-energetische Bestrahlungen
Method of beam controlling and apparatus for treatment of lesion by high energy radiation

(30) Priorité: 04.08.1992 FR 9209668
(43) Date de publication de la demande: 09.02.1994
(73) Titulaire: FRAMATOME, 92400 Courbevoie (FR)
(72) Inventeur: Kerjean, Joel, F-44800 Saint Herblain (FR)
(74) Mandataire: Dawidowicz, Armand

(56) Documents cités:
- EP-A- 0 056 552
- WO-A-92/08235
- DE-A- 2 351 450
- FR-A- 2 414 242
- US-A- 4 780 898
- US-A- 5 028 789

## Description

La présente invention est relative à un procédé pour le traitement de lésions par rayonnement à haute énergie.

Elle concerne plus particulièrement un procédé d'irradiation des lésions cérébrales par utilisation d'une source de rayons gamma associée à un collimateur.

L'invention est de même relative au dispositif de traitement mettant en oeuvre le procédé.

Dans les appareils à rayonnement gamma utilisés à ce jour, la destruction des lésions cérébrales traitées par neurochirurgie non intrusive est obtenue par la focalisation précise du rayonnement gamma sur la tumeur à traiter. Cette destruction doit faire l'objet d'une attention très particulière quant aux doses de rayonnement reçues et absorbées par les tissus sains.

Selon une première méthode connue, on utilise une mono source très intense dont le rayonnement est focalisé par un collimateur en un faisceau cylindrique étroit. L'intensité de ce mono faisceau oblige à appliquer un mouvement orbital continu autour de la lésion à traiter afin d'éviter la destruction des tissus intermédiaires traversés par le faisceau de très haute activité.

Le mouvement orbital continu est obtenu par motorisation des composants lourds qui sont le porte-source, le collimateur et les blindages associés.

Selon une seconde méthode connue, le risque de destruction des tissus sains est réduit par l'utilisation d'un ensemble de sources dont l'intensité individuelle ne peut pas entraîner des lésions aux tissus sains. En revanche, la focalisation individuelle de chacune de ces sources génère par sommation une dose intégrée suffisante pour le traitement de la lésion elle-même.

La méthode ci-dessus, telle que décrite par exemple dans le brevet US-A-4.780.898, nécessite l'implantation de sources radioactives de très petit diamètre, généralement de l'ordre du millimètre, et de leur collimateur respectif. Ces collimateurs, usinés dans des blindages du genre plomb, sont typiquement proches en nombre de 200 et leur usinage requiert des opérations longues et coûteuses. De plus, les sources sont éloignées du point de focalisation et une grande partie de leur énergie est convertie en chaleur par l'absorption des rayonnements par les composants eux-mêmes. Il en résulte que seule une faible partie des rayonnements traverse l'alésage servant de collimateur et constitue ainsi les seuls rayonnements utiles pour le traitement.

Cette méthode présente des inconvénients d'une part technologiques liés à la géométrie des composants et au mauvais rendement des sources et d'autre part économiques par l'absence d'optimisation de composants coûteux.

On a par ailleurs étudié un appareil visant à éliminer les défauts majeurs des méthodes citées ci-dessus en limitant la télécommande des composants les plus lourds et en diminuant le nombre de sources et leur dispositif de collimation associé.

Les recherches effectuées ont permis de maîtriser le déplacement relatif des sources et du patient ainsi que l'énergie nécessaire au traitement ponctuel des tumeurs.

Toutefois, ce traitement ponctuel, bien qu'il rende possible le traitement de la superficie d'une tumeur par déplacements relatifs, nécessite une durée d'irradiation relativement importante et ne satisfait pas pleinement les thérapeutes qui cherchent à obtenir une dose létale dans un volume bien délimité correspondant aux dimensions de la tumeur à traiter.

Afin de pallier ces inconvénients des procédés et dispositifs connus, la présente invention a pour objet un procédé de contrôle d'un faisceau de rayonnements gamma pour le traitement par irradiation et en particulier pour le traitement des lésions cérébrales, caractérisé en ce qu'on focalise, dans une première étape, les rayonnements d'une source selon un cheminement annulaire sensiblement divergent, puis qu'on écrête, dans une seconde étape, le faisceau annulaire ainsi créé des rayonnements obliques engendrés, et qu'on focalise, dans une ultime étape, le faisceau annulaire selon un cheminement annulaire sensiblement convergent jusqu'à l'obtention d'une tache focale volumique d'intensité et de dimension correspondant au traitement d'une lésion d'un volume donné.

Selon une caractéristique particulière, on choisit les caractéristiques géométriques des focalisations divergente et convergente de manière à obtenir un faisceau annulaire à émissions contrôlées qui permet l'optimisation de l'énergie de la source et l'obtention d'un gradient de décroissance plus élevé dans la zone d'occultation de la source.

L'invention concerne par ailleurs un appareil pour le traitement des lésions cérébrales par un faisceau de rayonnements gamma, ledit appareil comprenant au moins une source radioactive montée dans un canal d'un porte-source et un canal collimateur usiné dans un porte-collimateur, ainsi que des blindages,
caractérisé en ce que :
- un élément absorbant mâle ou aiguille, de profil divergent-convergent comportant une partie tronconique dont le sommet est au contact de la source, une partie médiane et une partie convergente, est disposé dans ledit canal collimateur,
- le canal collimateur comporte une surface conique divergente complémentaire de la partie divergente de l'élément absorbant mâle ainsi qu'une surface conique convergente complémentaire de la portion terminale convergente de l'élément absorbant mâle de manière à définir un espace annulaire divergent-convergent, et
- l'élément absorbant mâle comporte, dans la partie médiane, des ailettes assurant l'absorption des rayonnements périphériques parasites du faisceau divergent.

L'invention sera mieux comprise à l'aide de l'exemple non limitatif qui va être décrit et des figures qui y sont jointes et dans lesquelles :
la figure 1 est une coupe vue de face de l'ensemble source-collimateur-blindage d'un appareil de traitement auquel s'applique l'invention;
la figure 2 représente schématiquement trois dispositifs de focalisation, la figure 2a représentant l'art antérieur constitué par une source unique de haute intensité, la figure 2b représentant un ensemble source-collimateur non revendiqué pour irradiation ponctuelle, la figure 2c représentant un ensemble source-collimateur pour irradiation volumique;
la figure 3 est une vue en coupe d'un collimateur conforme à l'invention;
la figure 4 est une vue de détail selon une coupe de la partie médiane du collimateur de la figure 3; et
la figure 5 est une vue en coupe de la partie mâle du collimateur de la figure 3.

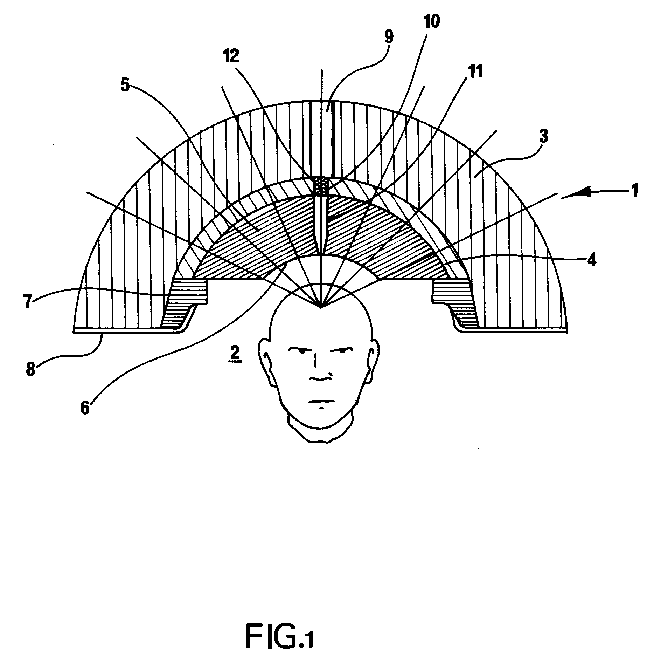

La figure 1 représente l'ensemble 1 comportant les porte-sources et porte-collimateurs d'un appareil pour le traitement des lésions cérébrales.

Cet ensemble 1 possède la forme d'une demi-sphère dont le côté ouvert 2 permet de laisser passer une table, non représentée, portant le patient.

Cet ensemble comporte un blindage extérieur 3 constitué par une forte épaisseur de plomb, une coquille 4 perforée constituant le porte-sources et un porte-collimateurs 5 de forte épaisseur définissant une surface intérieure sphérique 6.

Le porte-sources 4 et le porte-collimateurs 5 sont fixés de façon amovible au blindage 3 à l'aide d'une bague 7 maintenue par un flasque annulaire 8.

Cet ensemble est traversé de part en part par des canaux radiaux 9, 10 et 11 disposés de telle manière que leurs axes respectifs convergent vers le centre de la demi-sphère.

Ces canaux, dont un seul alignement est représenté sur la figure 1, répondent à trois fonctions différentes. A savoir, le canal 9 permet la manutention, le maintien et le blindage d'une source radioactive 12 notamment d'une source de Césium 137. La source 12 est positionée dans le canal 10 afin que sa surface affleure l'extrémité du canal collimateur 11.

Les canaux collimateurs 11 sont usinés dans le porte-collimateurs 5 constitué d'un matériau absorbant fortement les rayons gamma. Ces matériaux sont de manière préférentielle le plomb pur ou allié, le tungstène, l'uranium naturel, l'uranium appauvri en isotope 235, recouvert ou non d'un alliage inoxydable. On notera que les meilleurs résultats sont obtenus avec le couple Césium 137, Uranium (naturel ou appauvri).

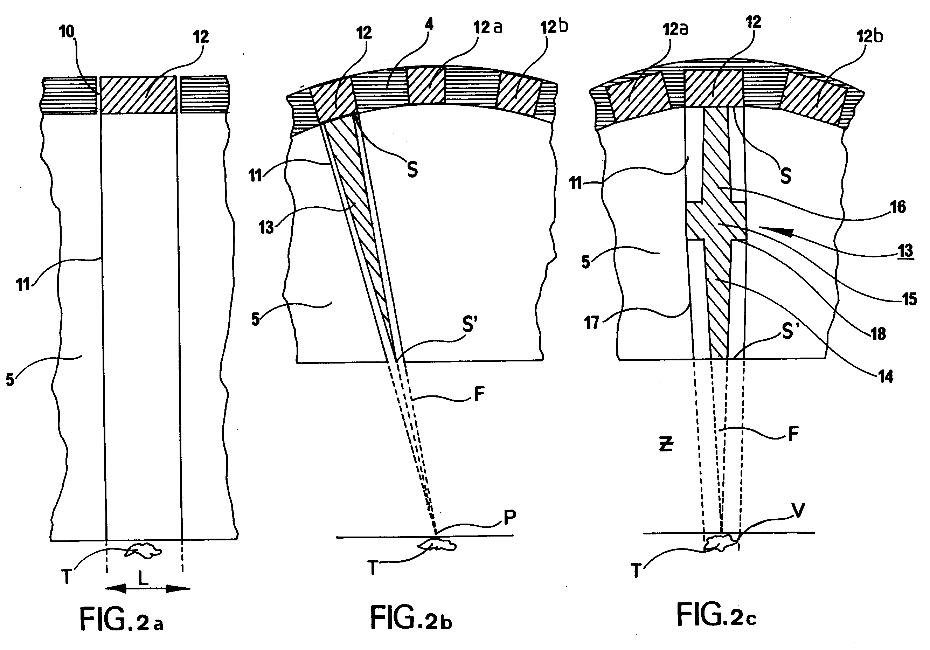

Comme représenté sur la figure 2a, l'art antérieur met en oeuvre des collimateurs de rayonnements gamma constitués d'une source unique, très souvent du Cobalt, associée à un simple alésage 11 dans lequel la source génère un faisceau de largeur utile L.

Cette disposition comporte des inconvénients dont le principal est constitué par la destruction des tissus sains avoisinants la lésion T et soumis à la même dose d'irradiation que cette dernière.

Cette disposition nécessite de plus l'utilisation de sources de haute intensité dont une partie importante des rayonnements émis est absorbée et transformée en chaleur dans le matériau du porte-collimateurs 5, ce qui lui confère un rendement mineur pour un coût excessif.

La figure 2b représente schématiquement un ensemble porte-sources 4 non revendiqué équipé de plusieurs sources 12, 12a, 12b de rayons gamma d'un diamètre de 10 millimètres environ. Ces sources 12, 12a, 12b sont disposées dans les canaux 10 du porte-sources 4 et sont ainsi positionnées au droit des canaux collimateurs 11.

Comme représenté sur la gauche de la figure 2b, le canal collimateur 11 est en forme de cône dont la base présente un diamètre proche du diamètre extérieur de la source 12 et dont le sommet possède une section de diamètre inférieur à ce dernier définissant ainsi, entre ces diamètres, un chemin convergent. La paroi conique de ce canal 11 focalise le rayonnement gamma en limitant l'absorption parasite par la masse du matériau porte-collimateurs 5. Cette focalisation est toutefois améliorée par la présence d'un élément absorbant mâle 13 de forme conique disposé au centre du canal 11 et au contact de la source 12 par sa base.

Cette géométrie définie par deux cônes de même sommet et dont les bases déterminent une surface utile émissive de forme annulaire S, procure un spectre de flux maximalisé au sommet S' commun aux deux cônes. On notera que le cône interne est défini par la surface de l'élément absorbant mâle ou aiguille 13 qui est de manière préférentielle de même matériau absorbant que le matériau du porte-collimateurs 5 recevant les canaux 11 dont la paroi définit le cône externe. Ce matériau est essentiellement de l'uranium soit sous sa forme naturelle soit sous sa forme appauvrie en isotope 235.

Le flux maximalisé F en sortie de canal 11 définit un faisceau annulaire focalisé de telle manière que ses génératrices convergent en un point P sur la cible constituée par la lésion T.

Il est évident que les diamètres respectifs du canal 11 et de l'élément absorbant mâle 13 sont calculés pour obtenir un point focal P le plus précis possible et dans le même temps une puissance maximale du faisceau annulaire sur ce point.

Cette réalisation est limitée par ce compromis car il est acquis que, si l'on augmente la section utile du faisceau annulaire pour exploiter le maximum d'intensité de la source et ainsi augmenter la puissance d'irradiation sur la lésion T, la convergence des rayonnements intervient dans un plan trop éloigné pour demeurer efficace.

La figure 2c montre enfin une réalisation conforme à l'invention dans laquelle la collimation du rayonnement gamma d'une source radioactive du type Césium 137 permet l'obtention d'une dose d'irradiation donnée dans un volume V défini.

En effet, le thérapeute cherche à réduire la durée importante d'irradiation nécessaire lors des traitements par faisceau ponctuel et vise à obtenir une dose létale dans un volume défini, assez réduit mais convenant pour les plus petites lésions à traiter.

Le traitement de ces lésions de l'ordre de quelques millimètres d'épaisseur impose évidemment que la décroissance de la dose soit aussi forte que possible au delà du volume traité.

La figure 2c montre de façon schématique un élément absorbant mâle 13 constituant le collimateur de l'invention permettant d'obtenir une tache focale volumique V ou volume cible assurant ainsi l'irradiation complète de la lésion.

Cet élément absorbant mâle 13 se compose d'une partie terminale 14 contigue aux sommets des deux cônes formés respectivement par la surface de ladite partie 14 et la surface 17 du canal collimateur 11, assurant ainsi la création et le contrôle d'un faisceau annulaire S' dont les génératrices convergent vers la lésion T et définissent le volume cible V.

Une partie médiane 15, comportant des ailettes en uranium 18, est disposée de telle manière que les ailettes 18 délivrent dans l'intervalle conique générateur du faisceau annulaire S' un flux maximal contrôlé et "tranquillisé", c'est-à-dire un flux écrêté des rayons périphériques obliques.

Une partie tronconique 16, dont la base est située à la partie médiane 15 et dont le sommet vient en appui sur la surface d'une source 12, définit avec la surface de la paroi du canal collimateur 11 un espace annulaire de forme divergente possédant une surface S de dimension supérieure à la surface au contact avec la partie médiane 15 munie d'ailettes 18.

La nouvelle géométrie de l'élément absorbant mâle 13, qui assure la création et le contrôle d'un faisceau utile sur une plage homogène ou un volume plus important, permet ainsi d'extraire une plus grande intensité de la source tout en augmentant le gradient de décroissance dans la zone d'occultation Z de la source.

Dans un mode de réalisation préféré de l'invention non représenté, il est possible de prévoir qu'au moins un des éléments pris dans le groupe suivant, partie terminale 14, partie médiane 15, partie tronconique 16 de l'élément absorbant mâle 13 est animé d'un mouvement relatif de manière à faire varier dans une plage importante le volume V de focalisation. Ainsi, à titre d'exemple, la partie terminale 14 de l'élément absorbant mâle 13 peut être constituée par un cône télescopique de manière à pouvoir être disposée en saillie du sommet du canal collimateur 17, en retrait de ce dernier ou en affleurement de ce dernier, faisant ainsi varier le faisceau obtenu.

Cette géométrie présente une forme générale de deux cônes assemblés par leurs bases et de sommets opposés définissant ainsi un ensemble présentant un profil divergent-convergent.

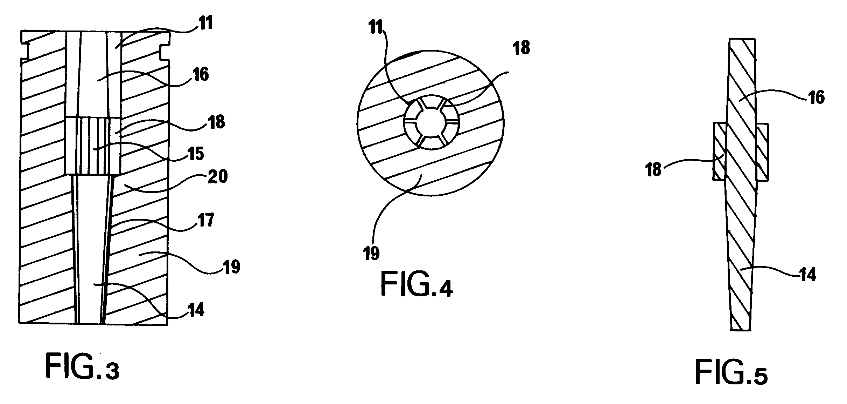

La figure 3 représente une réalisation conforme à la figure 2c dans laquelle le collimateur 13 est disposé dans un bloc 19 adaptable dans un blindage.

Le collimateur 13 et le bloc 19 sont en uranium naturel ou appauvri. Ils peuvent être complétés par une protection de plomb d'environ 1 millimètre pour améliorer la filtration de sortie.

La figure 3 montre en détail le collimateur 13 positionné dans les canaux collimateurs 11 et 17. A titre d'exemple, le canal collimateur 11 est constitué par un alésage de diamètre 19 mm, apte à coopérer avec une source de Césium 137 de 20 mm de diamètre. Le canal collimateur 17 est lui de forme tronconique et possède une base de 15 mm et un sommet de 10 mm.

La partie médiane 15 est positionnée dans l'alésage 11 avec un léger jeu et la partie inférieure des ailettes 18 repose sur un épaulement 20 pratiqué à la jonction des canaux collimateurs 11 et 17.

L'élément collimateur mâle 13 est lui-même dimensionné pour coopérer avec le profil des canaux collimateurs 11 et 17. A titre indicatif, l'élément collimateur mâle possède un diamètre médian de 10 mm alors que ses extrémités sont réduites à des diamètres de 7 ou 9 mm comme représenté sur la figure 5.

La figure 4 montre en coupe la répartition des ailettes en nombre de 6, mais il est évident que cette partie médiane 15 peut être équipée d'un nombre différent adapté à l'intensité recherchée du faisceau.

On décrira maintenant le procédé de fonctionnement de l'installation.

Après avoir prédimensionné la lésion T, le patient est positionné sur le site d'irradiation 2 desservi par l'ensemble porte-sources porte-collimateurs 1. Un clapet en matériau absorbant, non représenté, est alors effacé et le traitement de la lésion T s'effectue par un ensemble de sources-collimateurs fonctionnant comme suit.

La source 12 émet ses rayonnements gamma selon une diffusion azimutale. Ces rayonnements sont, dans cette première étape, focalisés par les parois du canal collimateur 11 pour produire un faisceau de rayonnements utiles. Cette focalisation est complétée par la focalisation obtenue grâce à la paroi de la partie tronconique 16 qui occulte faiblement la surface de la source 12, ce qui permet l'obtention d'un faisceau annulaire S s'amplifiant le long du cheminement annulaire défini par les parois sensiblement divergentes.

Cette amplification a pour conséquence un risque de création de rayonnements obliques parasites qui réduisent l'efficacité de la source et rendent difficilement contrôlable l'action du faisceau sur les tissus sains dans la zone d'occultation Z d'irradiation de la source.

Le faisceau annulaire amplifié pénètre alors dans la partie intermédiaire du canal collimateur 11 équipée des ailettes 18. Dans cette seconde étape, les ailettes agissent à la manière d'un tranquilliseur en écrêtant les rayonnements obliques proches des génératrices de l'hyperboloïde constituée par le faisceau annulaire.

Ce faisceau F écrêté dont les émissions sont contrôlées se présente sous la forme d'une veine annulaire type fluide à l'entrée d'une troisième étape de contrôle de sa focalisation.

Le faisceau, à mi-chemin de son parcours dans le collimateur, possède alors un maximum de puissance.

Dans la troisième étape, la partie terminale prend alors en charge le faisceau et le focalise en lui faisant suivre, grâce aux parois, un cheminement annulaire oblique de façon à ce que l'ensemble des génératrices constituant ce faisceau annulaire F converge jusqu'à une zone correspondant à l'emplacement de la lésion T où elles définissent un volume cible V de dimension et d'intensité nécessaires au traitement d'une lésion T d'un volume donné.

## Revendications

1. Procédé de contrôle d'un faisceau (F) de rayonnements gamma pour le traitement par irradiation et en particulier pour le traitement des lésions cérébrales,
caractérisé par les étapes suivantes :
- on focalise, dans une première étape, les rayonnements d'une source selon un cheminement annulaire sensiblement divergent,
- on écrête, dans une seconde étape, le faisceau annulaire ainsi créé des rayonnements obliques engendrés,
- on focalise, dans une ultime étape, le faisceau annulaire selon un cheminement annulaire sensiblement convergent jusqu'à l'obtention d'une tache focale volumique (V) d'intensité et de dimension correspondant au traitement d'une lésion (T) d'un volume donné.

2. Procédé suivant la revendication 1,
caractérisé en ce qu'on choisit les caractéristiques géométriques desdites focalisations divergente et convergente de manière à obtenir un faisceau annulaire à émissions contrôlées.

3. Procédé suivant la revendication 2,
caractérisé en ce qu'on écrête le faisceau annulaire des rayonnements obliques parasites au moyen d'ailettes (18) de tranquillisation en participant ainsi au contrôle du gradient de décroissance dans la zone (Z) d'occultation de la source (12).

4. Appareil pour le traitement des lésions cérébrales par un faisceau de rayonnements gamma, ledit appareil comprenant au moins une source radioactive (12) montée dans un canal (10) d'un porte-source (4) et un canal collimateur (11) usiné dans un porte-collimateur (5), ainsi que des blindages (3),
caractérisé en ce que :
- un élément absorbant mâle (13) ou aiguille, de profil divergent-convergent, comportant une partie tronconique (16) dont le sommet est au contact de la source (12), une partie médiane (15) et une partie terminale convergente (14), est disposé dans ledit canal collimateur (11),
- le canal collimateur (11) comporte une surface conique divergente complémentaire de la partie divergente (16) de l'élément absorbant mâle (13) ainsi qu'une surface conique convergente (17) complémentaire de la portion terminale convergente (14) de l'élément absorbant mâle (13) de manière à définir un espace annulaire divergent-convergent, et
- l'élément absorbant mâle (13) comporte dans la partie médiane (15) des ailettes (18) assurant l'absorption des rayonnements périphériques parasites du faisceau divergent.

5. Appareil suivant la revendication 4,
caractérisé en ce qu'au moins une des parties (14, 16) de l'élément absorbant mâle (13) est animée d'un mouvement relatif pour permettre une variation du début du faisceau et du volume de focalisation.

6. Appareil suivant l'une quelconque des revendications 4 et 5,
caractérisé en ce que les blindages (3), porte-sources (4), porte-collimateurs (5), éléments absorbants mâles (13) sont en un même matériau absorbant les rayonnements.

7. Appareil suivant la revendication 6,
caractérisé en ce que le matériau est de l'uranium naturel ou appauvri en isotope 235.

8. Appareil suivant l'une quelconque des revendications 4 à 7,
caractérisé en ce que le collimateur comporte une protection en plomb pour filtrer les rayonnements à leur sortie.

## Claims

1. Process for the control of a beam (F) of gamma rays for the treatment by irradiation and in particular for the treatment of cerebral lesions,
characterized by the following steps :
- focusing, in a first step, the radiations from a source according to an annular, substantially divergent path ;
- in a second step, removing from the so-created annular beam, the oblique radiations generated ;
- focusing, in an ultimate step, the annular beam according to an annular substantially convergent path until obtaining a focal volume spot (V) of an intensity and a dimension corresponding to the treatment of a lesion (T) of a given volume.

2. Process according to claim 1,
characterized in that the geometrical characteristics of said divergent and convergent focusing are chosen such as obtaining an annular beam of controlled emissions.

3. Process according to claim 2,
characterized in that the parasitic oblical radiations are removed from the annular beam by means of tranquillisation fins (18), thus contributing to the control of the gradient of decrease in the zone (Z) of occultation of the source (12).

4. Apparatus for the treatment of cerebral lesions by a beam of gamma radiations said apparatus comprising at least one radioactive source (12) mounted in a channel (10) of a source carrier (4) and a collimator channel (11) machined in a collimator carrier (5), as well as shields (3),
characterized in that :
- a male absorbent element (13) or needle, of divergent-convergent profile, having a trunconical part (16) the tip of which is in contact with the source (12), a median part (15) and a convergent terminal part (14), is disposed in said collimator channel (11),
- the collimator channel (11) contains a divergent conical surface complementary to the divergent part (16) of the absorbent male element (13) as well as a convergent conical surface (17) complementary of the convergent terminal portion (14) of the male absorbent element (13) in such a way to define an annular divergent-convergent space, and
- the absorbent male element (13) is provided in the median part (15) with the fins (18) assuming the absorption of the parasitic peripheral radiations of the divergent beam.

5. Apparatus according to claim 4,
characterized in that at least one of the parts (14, 16) of the male absorbent element (13) is moved in a relative movement in order to allow for a variation of the beginning of the beam and of the volume of focusing.

6. Apparatus according to anyone of claims 4 and 5,
characterized in that the shields (3), source carrier (4), collimator carrier (5), male absorbent elements (13) are of a same material which absorbs the radiations.

7. Apparatus according to claim 6,
characterized in that the material is natural uranium of uranium impoverished in isotope 235.

8. Apparatus according to anyone of claims 4 to 7,
chacaracterized in that the collimator channel comprises a lead protective to filter the radiations ot their outlet.

## Patentansprüche

1. Verfahren zur Regulierung eines Gammastrahlenbündels (F) zur Behandlung durch Bestrahlung, insbesondere zur Behandlung von zerebralen Schädigungen,
**gekennzeichnet** durch die folgenden Schritte:
- in einem ersten Schritt werden die Strahlen einer Strahlungsquelle entsprechend einem weitgehend divergierenden ringförmigen Verlauf fokussiert,
- in einem zweiten Schritt werden dem so erzeugten Strahlenbündel die entstandenen Schrägstrahlen entzogen,
- in einem letzten Schritt wird das ringförmige Strahlenbündel entsprechend einem weitgehend konvergierenden ringförmigen Verlauf bis zur Erzielung eines volumenbezogenen Brennflecks (V) mit einer Intensität und Abmessung fokussiert, die der Behandlung einer Schädigung (T) mit einem gegebenen Volumen entsprechen.

2. Verfahren nach Anspruch 1,
**dadurch gekennzeichnet,** daß die geometrischen Eigenschaften der besagten divergierenden und konvergierenden Fokussierung so gewählt werden, daß ein ringförmiges Strahlenbündel mit kontrollierten Emissionen erzielt wird.

3. Verfahren nach Anspruch 2,
**dadurch gekennzeichnet,** daß dem ringförmigen Strahlenbündel die Schrägstörstrahlen anhand von Beruhigungsflügeln (18) entzogen werden, wodurch zur Kontrolle des Abnahmegradienten in der Ausblendungszone (Z) der Strahlungsquelle (12) beigetragen wird.

4. Gerät zur Behandlung von zerebralen Schädigungen durch ein Gammastrahlenbündel, wobei das besagte Gerät mindestens eine radioaktive Strahlungsquelle (12) umfaßt, die in einem Kanal (10) eines Strahlerträgers (4) angebracht ist, und einen Kollimatorkanal (11), der in einen Kollimatorträger (5) eingearbeitet ist, sowie Abschirmungen (3) umfaßt,
**dadurch gekennzeichnet**, daß:
- in dem besagten Kollimatorkanal (11) ein Einsteckabsorbierelement (13) oder eine Nadel mit divergierend-konvergierendem Profil angeordnet ist, das einen kegelstumpfartigen Teil (16), dessen Spitze mit der Strahlungsquelle (12) in Berührung steht, einen Mittelteil (15) und einen konvergierenden Endteil (14) umfaßt,
- der Kollimatorkanal (11) eine divergierende konische Fläche passend zum divergierenden Teil (16) des Einsteckabsorbierelements (13) sowie eine konvergierende konische Fläche (17) passend zum konvergierenden Endteil (14) des Einsteckabsorbierelements (13) umfaßt, so daß ein divergierend-konvergierender ringförmiger Zwischenraum gebildet wird, und
- das Einsteckabsorbierelement (13) im Mittelteil (15) Flügel (18) umfaßt, um die Absorption der Randstörstrahlen des divergierenden Strahlenbündels zu bewirken.

5. Gerät nach Anspruch 4,
**dadurch gekennzeichnet,** daß mindestens einer der Teile (14, 16) des Einsteckabsorbierelements (13) in einer relativen Bewegung angetrieben wird, um eine Veränderung des Beginns des Strahlenbündels und des Fokussierungsvolumens zu ermöglichen.

6. Gerät nach einem der Ansprüche 4 und 5,
**dadurch gekennzeichnet,** daß die Abschirmungen (3), Strahlerträger (4), Kollimatorträger (5) und Einsteckabsorbierelemente (13) aus einem gleichen die Strahlen absorbierenden Material bestehen.

7. Gerät nach Anspruch 6,
**dadurch gekennzeichnet,** daß das Material natürliches oder an Isotop 235 abgereichertes Uran ist.

8. Gerät nach einem der Ansprüche 4 bis 7,
**dadurch gekennzeichnet,** daß der Kollimator einen Schutz aus Blei umfaßt, um die Strahlen bei ihrem Austritt zu filtern.
